Europäisches Patentamt

(19) European Patent Office

Office européen des brevets

(11) Publication number: **0 034 428**
**B1**

(12) **EUROPEAN PATENT SPECIFICATION**

(45) Date of publication of patent specification: **28.09.83**

(21) Application number: **81300416.5**

(22) Date of filing: **02.02.81**

(51) Int. Cl.³: **C 07 B 19/00,**
**C 07 C 51/487,**
**C 07 C 61/40, C 07 C 87/28**

(54) A method of resolving optical isomers of 2,2-dimethyl-3-(2,2-dichlorovinyl) cyclopropanecarboxylic acid and a novel salt used in this method.

(30) Priority: **31.01.80 JP 11313/80**

(43) Date of publication of application:
**26.08.81 Bulletin 81/34**

(45) Publication of the grant of the patent:
**28.09.83 Bulletin 83/39**

(84) Designated Contracting States:
**CH DE FR GB IT LI NL**

(56) References cited:
**EP - A - 0 006 187**
**DE - A - 2 251 097**
**FR - A - 2 427 321**
**GB - A - 2 008 589**

(73) Proprietor: **SUMITOMO CHEMICAL COMPANY, LIMITED**
**15 Kitahama 5-chome Higashi-ku**
**Osaka-shi Osaka-fu (JP)**

(72) Inventor: **Suzuki, Yukio**
**10-2-241, Sonehigashinocho 2-chome**
**Toyonaka-shi (JP)**

(74) Representative: **Tubby, David George et al,**
**MARKS & CLERK 57-60 Lincoln's Inn Fields**
**London WC2A 3LS (GB)**

Courier Press, Leamington Spa, England.

# O 034 428

### A method of resolving optical isomers of 2,2-dimethyl-3-(2,2-dichlorovinyl) cyclopropanecarboxylic acid and a novel salt used in this method

This invention relates to a method of resolving optical isomers of *cis*-2,2-dimethyl-3-(2,2-dichlorovinyl)cyclopropanecarboxylic acid [hereinafter 2,2-dimethyl-3-(2,2-dichlorovinyl)cyclopropanecarboxylic acid is referred to briefly as "DV acid"].

DV acid is the acid part of the molecules of Cypermethrin and Permethrin which have recently attracted keen attention as agricultural insecticides and a disinfectant insecticides. Since DV acid has two asymmetric carbon atoms in its cyclopropane ring, it has four stereoisomers. Of the four stereoisomers of Permethrin and Cypermethrin, those having the acid part in the (1S, *cis*)- or (1S, *trans*)-form possess little insecticidal activity, whereas those having the acid part in the (1R, *cis*)- or (1R, *trans*)-form are effective. Although the difference in insecticidal activity between the esters of the (1R, *cis*)-acid and the (1R, *trans*)-acid depends on the target insect species, the former esters show a higher activity against principal agricultural pests.

The synthesis of (1R, *cis*)-DV acid from optically active chrysanthemic acid is known [M. Elliot *et al.*, Nature, *244*, 456 (1973)]. This method, however, uses a starting material which is expensive or has limited availability on a commercial basis; moreover, the method involves many preparative steps, resulting in a low yield of the intended product.

Another method of synthesizing DV acid rich in the (1R, *cis*)-form uses an asymmetric copper catalyst (Japanese Patent Publication No. 46,835/1978). One of the disadvantages of this method is that a number of steps are involved in preparing the catalyst.

On the other hand, there are known methods of preparing DV acid by optical resolution. Firstly, as to *trans*-DV acid, the known optical resolution method employs $\underline{D}$-(—)-threo-1-*p*-nitrophenyl-2-(*N,N*-dimethylamino)propane-1,3-diol, (+)-erythro-1,2-diphenyl-2-hydroxyethylamine and (—)-$\beta$-dimethyl-amino-$\alpha,\alpha$-dimethyl-$\beta$-phenethyl alcohol [P. E. Burt *et al.*, Pestic. Sci., 1974, *5*, 791—799; Japanese Patent Application Kokai (i.e. as laid open to public inspection No. 36,441/1976 and No. 143,647/1976].

Secondly, as to *cis*-DV acid, there are known optical resolution methods which employ (+)-$\alpha$-methylbenzylamine or (—)-quinone (P. E. Burt *et al.*, *loc. cit.*; British Patent Application No. 2,008,589A). In the former case, because of the low optical purity of the resulting (1R, *cis*)-DV acid, recrystallization of the (+)-$\alpha$-methylbenzylamine salt must be repeated several times and also the method lacks reproducibility. In the latter case, being one of the natural alkaloids,(—)-quinine is irregular in its commercial supply and demand, lacks the chemical stability which is necessary for its recovery and reuse in an industrial process, and has, above all, the great disadvantage of a much higher price than synthetic optical resolution agents.

We have now found that optically active 1-phenyl-2-(*p*-tolyl)ethylamine (hereinafter referred to as PTE), which is one of the most readily available of the many synthetic amines which are optically active, may be used industrially as an optical resolution agent for the *cis*-DV acid.

Thus, the present invention provides a method of resolving (1R, *cis*)- or (1S, *cis*)-DV acid from a DV acid containing at least 70% of the *cis* acid, by adding to said DV acid (+)-PTE or (—)-PTE in an amount not greater than equimolar with respect to said DV acid, separating and, if necessary, purifying the resulting diastereomer salt, and then decomposing the salt to free the (1R, *cis*) or (1S, *cis*)-DV acid, respectively.

The process of the invention can be adapted to separate either (1R, *cis*)-DV acid [by using (+)-PTE] or (1S, *cis*)-DV acid [by using (—)-PTE] from a DV acid mixture. Since (1R, *cis*)-DV acid is the isomer having the greater commercial value, the invention is hereafter described with reference to the separation of this isomer. It will, however, be appreciated that the conditions described below also apply *mutatis mutandis* to the separation of the (1S, *cis*) isomer.

The diastereomer salts produced, *viz.* (+ or —)-1-phenyl-2-(*p*-tolyl)ethylammonium (1R or 1S, *cis*)-2,2-dimethyl-3-(2,2-dichlorovinyl)cyclopropanecarboxylate are new compounds and also form part of the present invention.

First, to a DV acid containing 70% or more of the *cis*-form, is added an equimolar or lesser amount of (+)-PTE in order to form a salt. The use of the amine in an amount greater than equimolar with respect to the acid will cause a decrease in the optical purity of the DV acid in the salt. In view of the optical purity and yield of the salt, the amount of (+)-PTE added is preferably in the range of from 0.5 to 0.8 mole per mole of DV acid and its optical purity is preferably 90% or higher, the higher the better.

The DV acid used as starting material should contain 70% or more, preferably 80% or more, of the *cis*-form, and the higher the *cis* content the better.

The salt formation is preferably carried out in a solvent. It can also be carried out in the presence of another achiral organic or inorganic base in an amount of 0.5 mole or less (per mole of the DV acid used as starting material). The solvent may be freely selected from inert solvents other than those acidic or basic solvents which will interfere with salt formation.

The salt thus formed is then allowed to precipitate in the solvent to separate the crystals from the mother liquor. Heating is not essential but in general if the temperature of the solution is raised before or after salt formation and then reduced to a temperature at which the salt crystals fully precipitate better separation of the crystals from the mother liquor is achieved. In this case, the solvent may be freely selected from inert solvents other than those acidic or basic solvents which interfere with the salt formation. In view of handling, a lower alcohol (e.g. methanol or ethanol) or a mixture of a lower alcohol and water is preferred.

If necessary, the optical purity or chemical purity of the salt thus formed can be improved by recrystallization or the like treatment using a solvent which may be the same as or different from that used in depositing the salt.

The salt isolated as crystals is then separated into DV acid and PTE by the action of an acid or a base. The acid to be used should be an acid such as, for example, a mineral acid (e.g. hydrochloric acid or sulphuric acid) which is stronger than the DV acid, and the base to be used should be a base such as, for example, an inorganic base (e.g. sodium hyroxide or potassium hydroxide) which is a stronger base than PTE. Such an acid or base is used in an amount equimolar or greater with respect to the salt. Since the solubility of inorganic acid salts of PTE in water is small, it is preferable to decompose the salt with an inorganic base and, after removing the PTE by extraction with an organic solvent, to set free the DV acid with an excess of an inorganic acid and recover it. If the DV acid used as starting material contains some of the *trans* isomer, the DV acid obtained will contain some *trans* isomer, which can be removed, if necessary, by recrystallization process in the form of the intermediate DV acid salt or the separated free DV acid.

The invention is illustrated below with reference to the following Examples and Comparative Examples, in which "*cis*-DV acid" and "*trans*-DV acid" mean the *cis* and *trans* isomers containing substantially no other isomers; and the optical purity or the optical isomer ratio was determined by converting the DV acid into a d-2-octanol ester and analyzing by gas chromatography [M. Horiba *et al.*, Agr. Biol. Chem., *41*, 581 (1977)].

Example 1

To a solution of 10.45 g (50 mmoles) of *cis*-DV acid in 70 g of ethanol were added dropwise, under reflux, 6.87 g (32.5 mmoles) of (+)-PTE. Upon cooling the mixture slowly to about 60°C, crystals were deposited. After cooling the mixture to 26°C, the crystals were collected by filtration and then washed with 50 g of ethanol. The yield of crystals was 7.59 g (72.2% based on one-half of the charged DV acid); $[\alpha]_D^{21}+39.8°$ (methanol, c=1.07); melting at 156—159°C.

7 g portion of these crystals was added to a mixture of 20 cc of toluene and 50 cc of a 2% solution of sodium hydroxide. The resulting mixture was stirred for one hour at 40°C and allowed to settle into two layers. The aqueous layer was thoroughly mixed with 10 cc of toluene and allowed to settle into two layers. The aqueous layer was thoroughly mixed with 10 cc of 10% hydrochloric acid and 20 cc of toluene and allowed to settle into two layers. The aqueous layer was extracted with 20 cc of toluene. The toluene layers were combined, washed with water and concentrated to give 3.45 g of (1R, *cis*)-DV acid; $[\alpha]_D^{24.5}+31.86°$ (chloroform, c=3.44); optical purity 90.0%.

Example 2

To a solution of 10.45 g (50 mmoles) of *cis*-DV acid in 51 g of 80% ethanol, were added dropwise at 20—30°C 6.87 g (32.5 mmoles) of (+)-PTE. The reaction system became a highly viscous slurry. After completing dissolution by heating, the resulting solution was cooled slowly with stirring (deposition of crystals at about 58°C) and the crystals were collected by filtration at 23°C. The crystals were washed with 60 cc of 80% ethanol and dried to give 8.73 g (83.2% based on one-half of the charged DV acid) of the intended salt, which was subsequently decomposed as in Example 1 to give (1R, *cis*)-DV acid, $[\alpha]_D^{23}+24.2°$ (chloroform, c=4.16).

Example 3

To a solution of 10.45 g (50 mmoles) of *cis*-DV acid in 73.15 g of ethanol were added dropwise, under reflux, 8.45 g (40 mmoles) of (+)-PTE. The solution was slowly cooled (deposition of crystals at about 56°C) and stirred at 21.5°C for 90 minutes. The crystals were collected by filtration, washed with 150 cc of ethanol, and dried to give 8.46 g (80.9% based on one-half of the charged DV acid) of the intended salt, which was then decomposed to yield (1R, *cis*)-DV acid $[\alpha]_D^{24}+24.7°$ (chloroform, c=3.44).

Example 4

To a solution of 10.45 g (50 mmoles) of *cis*-DV acid in 52.25 g of methanol, were added dropwise, under reflux, 5.56 g (25 mmoles) of (+)-PTE. After completion of the dropwise addition, the reaction solution was slowly cooled (deposition of crystals at about 47°C) and filtered at 23°C to collect the crystals. The crystals were washed with 20 cc of methanol and dried to give 5.38 g (51.5% based on one-half of the charged DV acid) of the intended salt, which, on decomposition, yielded (1R, *cis*)-DV acid, $[\alpha]_D^{24}+28.7°$ (chloroform, c=4.41).

Example 5

To a solution of 10.45 g (50 mmoles) of *cis*-DV acid in 52.25 g of carbon tetrachloride, were added dropwise, under reflux, 5.56 g of (+)-PTE. After completion of the addition, the reaction solution was cooled slowly (deposition of crystals at about 65°C), stirred at 55°C for one hour, and filtered at the same temperature. The salt was washed with 30 cc of carbon tetrachloride and dried to give 6.24 g (59.4% based on one-half of the charged DV acid) of the salt which yielded (1R, *cis*)-DV acid, $[\alpha]_D^{23}+15.1°$ (chloroform, c=6.0).

Example 6

To 137.8 g of ethanol, were added 24.35 g of a salt of crude (1R, *cis*)-DV acid {$[\alpha]_D^{23}+24.58°$ (chloroform, c=3.10); 72.5% optical purity} and (+)-PTE, and then the salt was dissolved under reflux. The resulting solution was slowly cooled with stirring (deposition of crystals at about 71°C) and filtered at 27°C. The crystals were washed with 50 g of 60% ethanol and dried, to give 19.37 g (79.5% recrystallization yield) of crystals which yielded (1R, *cis*)-DV acid having an optical rotation $[\alpha]_D^{22.5}$ of +33.0° (chloroform, c=3.77) and an optical purity of 97.4%.

Example 7

To a solution of 10.46 g (50 mmoles) of DV acid (containing the *cis*-form and *trans*-form in a ratio of 80:20) in 73.22 g of ethanol, were added dropwise at 22—25°C 6.87 g of (+)-PTE. After completion of the addition, the solution was stirred continuously at 25°C and gradually deposited crystals. After 3 hours stirring, the mixture was filtered and the salt was washed with 30 cc of ethanol and dried to give 4.20 g (40.0% based on one-half of the charged DV-acid) of a salt which yielded DV acid of the following analysis: 87.2% (1R, *cis*); 5.5% (1S, *cis*); 2.4% (1R, *trans*); 4.9% (1S, *trans*); $[\alpha]_D^{21}+26.5°$ (chloroform, c=2.04).

Example 8

To a solution of 10.46 g (50 mmoles) of DV acid (containing the *cis*-form and *trans*-form in a ratio of 80:20) in 73.22 g of 80% ethanol, were added dropwise at room temperature 6.87 g of (+)-PTE. After completion of the addition, the precipitated crystals were dissolved by elevating the temperature and the solution was then cooled with stirring (deposition of crystals at 42°C). The crystals were collected by filtration at 23°C, washed with 30 cc of 80% ethanol, and dried to give 7.15 g (68.0% based on one-half of the charged DV acid) of a salt having a melting point of 145—152°C and an optical rotation $[\alpha]_D^{21}$ of +44.4° (methanol, c=1.76). The DV acid obtained from this salt showed the following analysis: 79.0% (1R, *cis*): 11.7% (1S, *cis*; 3.9% (1R, *trans*); 5.9% (1S, *trans*); $[\alpha]_D^{21}+24.2°$ (chloroform, c=2.06).

Example 9

To a solution of 10.46 g (50 mmoles) of DV acid (containing the *cis*-form and *trans*-form in a ratio of 70:30) in 73.22 g of ethanol, were added dropwise at room temperature 6.87 g of (+)-PTE. After completion of the addition, the mixture was cooled with stirring to 5° to 7°C, when crystals separated out. The mixture was then stirred for a further four hours and filtered. The crystals were washed with 30 cc of ethanol and dried to give 4.43 g (42.2% based on one-half of the charged DV acid) of the salt. The DV acid obtained from this salt showed the following analysis: 47.4% (1R, *cis*); 29.3% (1S, *cis*); 13.0% (1R, *trans*); 10.3% (1S, *trans*); $[\alpha]_D^{21}+8.64°$ (chloroform, c=2.08).

Comparative Example 1

To a solution of 14.6 g (70 mmoles) of *cis*-DV acid in 250 cc of benzene, were added dropwise, under reflux, 8.47 g (70 mmoles) of (+)-$\alpha$-methylbenzylamine. After completion of the addition, the mixture was cooled with stirring (deposition of crystals at 43°C) and filtered at 20°C. The crystals were washed with 30 cc of benzene to give 21.47 g (93.1% based on the charged DV acid) of crystals. The *cis*-DV acid obtained by decomposition of these crystals showed an optical rotation $[\alpha]_D^{24}$ of −0.62° (chloroform, c=5.78).

Comparative Example 2

To a solution of 2.67 g (12.8 mmoles) of *cis*-DV acid in 45.7 cc of benzene, which was kept at 50°C, were added dropwise 1.55 g (12.8 mmoles) of (+)-$\alpha$-methylbenzylamine. After addition of 35.5 cc of benzene, the reaction mixture was left standing at 50—60°C for 2 hours, then at room temperature overnight, and filtered at 25°C. The crystals were washed with 10 cc of benzene to give 3.84 g (91.1% based on the charged DV acid) of crystals. The *cis*-DV acid obtained by the decomposition of these crystals showed an optical rotation $[\alpha]_D^{25}$ of −0.58° (chloroform, c=5.53).

Comparative Example 3

To a solution of 10.45 g (50 mmoles) of *cis*-DV acid in 52 g of 80% ethanol, were added dropwise at 20—25°C 5.56 g (32.5 mmoles of (−)-$\alpha$-(1-naphthyl))ethylamine. After completion of the addition, crystals separated out, forming a viscous slurry which changed into a clear solution upon heating to 45°C. The solution was cooled slowly (deposition of crystals at 36°C) and filtered at 23.5°C. The crystals were washed with 50 cc of 80% ethanol and dried to give 5.59 g (29.4% based on the charged DV acid) of a salt. The *cis*-DV acid in the salt showed an optical rotation $[\alpha]_D^{23}$ of $-1.46°$ (chloroform, c=5.56).

Comparative Example 4

Optical resolution was performed in the same manner as in Example 2, except that *trans*-DV acid was used in place of the *cis*-DV acid. There was obtained 7.81 g (37.2% based on the charged DV acid) of a salt. The *trans*-DV acid obtained from the salt showed an optical rotation $[\alpha]_D^{23.5}$ of $+0.77°$ (chloroform, c=4.73).

Comparative Example 5

To a solution of 10.46 g (50 mmoles) of DV acid (containing the *cis*-form and *trans*-form in a ratio of 43.4:56.6) in 73.22 g of ethanol, were added dropwise at room temperature 6.87 g (32.5 mmoles) of (+)-PTE. No crystallization took place on stirring the solution or even on the further dropwise addition of 3.70 g (17.5 mmoles) of (+)-PTE. When 18.3 g of water was added, crystals separated out. The temperature was raised to 38°C to dissolve the crystals and the resulting solution was then cooled slowly with stirring (deposition of crystals at 34°C) and filtered at 25°C. The crystals were washed with 30 cc of ethanol and dried to give 9.14 g (43.5% based on the charged DV acid) of a salt. The DV acid obtained from the salt showed the following analysis: 19.9% (1R, *cis*); 15.3% (1S, *cis*); 33.3% (1R, *trans*); 31.5% (1S, *trans*); $[\alpha]_D^{21}+0.72°$ (chloroform, c=1.67).

## Claims

1. A method of resolving (1R, *cis*)- or (1S, *cis*)-2,2-dimethyl-3-(2,2-dichlorovinyl)cyclopropanecarboxylic acid from a 2,2-dimethyl-3-(2,2-dichlorovinyl)-cyclopropanecarboxylic acid containing at least 70% of the *cis* acid, by adding to said 2,2-dimethyl-3-(2,2-dichlorovinyl)cyclopropanecarboxylic acid (+) or (−)-1-phenyl-2-(p-tolyl)ethylamine in an amount not greater than equimolar with respect to said 2,2-dimethyl-3-(2,2-dichlorovinyl)cyclopropanecarboxylic acid, separating and, if necessary, purifying the resulting diastereomer salt, and then decomposing the salt to free the (1R, *cis*)- or (1S, *cis*)-2,2-dimethyl-3-(2,2-dichlorovinyl)cyclopropanecarboxylic acid, respectively.

2. A method according to Claim 1 for the resolution of the (1R, *cis*) acid, in which (+)-1-phenyl-2-(p-tolyl)ethylamine is used.

3. A method according to Claim 1 or Claim 2, wherein 2,2-dimethyl-3-(2,2-dichlorovinyl)cyclopropanecarboxylic acid containing 80% or more of the *cis*-form is used.

4. A method according to any preceding Claim, wherein from 0.5 to 0.8 mole of the 1-phenyl-2-(p-tolyl)ethylamine is used per mole of *cis*-2,2-dimethyl-3-(2,2-dichlorovinyl)cyclopropanecarboxylic acid.

5. A method according to any preceding Claim, wherein the reaction of 2,2-dimethyl-3-(2,2-dichlorovinyl)cyclopropanecarboxylic acid with 1-phenyl-2-(p-tolyl)ethylamine, and the precipitation of the diastereomer salt are carried out in the same solvent.

6. A method according to Claim 5, wherein the solvent is a lower alcohol.

7. A method according to Claim 6, wherein the lower alcohol is methanol or ethanol.

8. A method according to any preceding Claim, wherein the purification of the precipitated diastereomer salt is carried out by recrystallization.

9. A method according to any preceding Claim, wherein decomposition of said diastereomer salt is effected with a base or an acid.

10. (+ or −)-1-Phenyl-2-(p-tolyl)ethylammonium (1R, or 1S, *cis*)-2,2-dimethyl-3-(2,2-dichlorovinyl)cyclopropanecarboxylate.

## Patentansprüche

1. Verfahren zur Trennung von (1S,cis)-oder (1S,cis)-2,2-Dimethyl-3-(2,2-dichlorovinyl)-cyclopropancarbonsäure von 2,2-Dimethyl-3-(2,2-dichlorvinyl)-cyclopropancarbonsäure mit mindestens 70% an cis-Säure durch Zusatz einer, bezogen auf die 2,2-Dimethyl-3-(2,2-dichlorvinyl)-cyclopropancarbonsäure, höchstens äquimolaren Menge (+)- oder (−)-1-Phenyl-2-(p-tolyl)-ethylamin zur der 2,2-Dimethyl-3-(2,2-dichlorvinyl)-cyclopropancarbonsäure, Trennung und, erforderlichenfalls, Reinigung des erhaltenen Diastereomerensalzes und anschließende Zersetzung des Salzes zu der freien (1R,cis)- bzw. (1S,cis)-2,2-Dimethyl-3-(2,2-dichlorvinyl)-cyclopropancarbonsäure.

2. Verfahren nach Anspruch 1 zur Abtrennung der (1R,cis)-Säure unter Verwendung von (+)-1-Phenyl-2-(p-tolyl)-ethylamin.

3. Verfahren nach Ansprüchen 1 oder 2, dadurch gekennzeichnet, daß man eine 2,2-Dimethyl-3-(2,2-dichlorvinyl)-cyclopropancarbonsäure mit 80% oder mehr an cis-Form verwendet.

4. Verfahren nach einem der vorhergehenden Ansprüche, dadurch gekennzeichnet, daß man pro Mol cis-2,2-Dimethyl-3-(2,2-dichlorvinyl)-cyclopropancarbonsäure 0,5 bis 0,8 Mol 1-Phenyl-2-(p-tolyl)-ethylamin verwendet.

5. Verfahren nach einem der vorhergehenden Ansprüche, dadurch gekennzeichnet, daß die Umsetzung von 2,2-Dimethyl-3-(2,2-dichlorovinyl)-cyclopropancarbonsäure mit 1-Phenyl-2-(p-tolyl)-ethylamine und die Ausfällung des Diasteromerensalzes im selben Lösungsmittel erfolgen.

6. Verfahren nach Anspruch 5, dadurch gekennzeichnet, daß man also Lösungsmittel einen niederen Alkohol verwendet.

7. Verfahren nach Anspruch 6, dadurch gekennzeichnet, daß man als niederen Alkohol Methanol oder Ethanol verwendet.

8. Verfahren nach einem der vorhergehenden Ansprüche, dadurch gekennzeichnet, daß man die Reinigung des ausgefällten Diastereomerensalzes durch Umkrisallisierung durchführt.

9. Verfahren nach einem der vorhergehenden Ansprüche, dadurch gekennzeichnet, daß die Zersetzung des Diasteromerensalzes mittels einer Base oder einer Säure erfolgt.

10. (+)- oder (—)-1-Phenyl-2-(p-tolyl)-ethylammonium-(1R,cis)- oder -(1S,cis)-2,2-dimethyl-3-(2,2-dichlorvinyl)-cyclopropancarboxylat.

## Revendications

1. Procédé pour l'obtention d'acide 2,2-diméthyl-3-(2,2-dichlorovinyl)-cyclopropane-carboxylique (1R,cis) ou (1S,cis) par dédoublement d'un acide 2,2-diméthyl-3-(2,2-dichlorovinyl)-cyclopropane-carboxylique qui contient au moins 70% de l'acide cis, par addition, à cet acide 2,2-diméthyl-3-(2,2-dichlorovinyl)-cyclopropane-carboxylique, de 1-phényl-2-(p-tolyl)-éthylamine-(+) ou -(=) dans une quantité qui ne dépasse pas la proportion équimolaire par rapport à l'acide 2,2-diméhyl-3-(2,2-dichlorovinyl)-cyclopropane-carboxylique, séparation et, si nécessaire, purification du sel diastéréo-isomère résultant, puis décomposition du sel pour libérer l'acide 2,2-diméthyl-3-(2,2-dichlorovinyl)-cyclopropane-carboxylique-(1R,cis) ou (1S,cis) respectivement.

2. Procédé selon la revendication 1 pour l'obtention de l'acide (1R,cis) par dédoublement, caractérisé en ce qu'on utilise la 1-phényl-2-(p-tolyl)-éthylamine-(+).

3. Procédé selon la revendication 1 ou 2, caractérisé en ce qu'on utilise un acide 2,2-diméthyl-3-(2,2-dichlorovinyl)-cyclopropane-carboxylique qui contient 80% de la forme cis ou davantage.

4. Procédé selon l'une quelconque des revendications 1 à 3, caractérisé en ce qu'on utilise 0,5 à 0,8 mol de la 1-phényl-2-(p-tolyl)-éthylamine par mol d'acide 2,2-diméthyl-3-(2,2-dichlorovinyl)-cyclopropane-carboxylique-cis.

5. Procédé selon l'une quelconque des revendications 1 à 4, caractérisé en ce que la réaction de l'acide 2,2-diméthyl-3-(2,2-dichlorovinyl)-cyclopropane-carboxylique avec la 1-phényl-2-(p-tolyl)-éthylamine et la précipitation du sel diastéréo-isomère sont effectuées dans le même solvant.

6. Procédé selon la revendication 5 caractérisé en ce que le solvant est un alcool inférieur.

7. Procédé selon la revendication 6, caractérisé en ce que l'alcool inférieur est le méthanol ou l'éthanol.

8. Procédé selon l'une quelconque des revendications 1 à 7, caractérisé en ce que la purification du sel diastéréo-isomère précipité est effectuée par recristallisation.

9. Procédé selon l'une quelconque des revendications 1 à 8, caractérisé en ce que la décomposition du sel diastéréo-isomère est effectuée avec une base ou un acide.

10. 2,2-diméthyl-3-(2,2-dichlorovinyl)-cyclopropane-carboxylate-(1R ou 1S,cis) de 1-phényl-2-(p-tolyl)-éthylammonium-(+) ou -(—).